(19) Europäisches Patentamt

European Patent Office

Office européen des brevets

(11) **EP 1 494 028 A1**

(12) **EUROPEAN PATENT APPLICATION**

(43) Date of publication:
**05.01.2005 Bulletin 2005/01**

(51) Int Cl.[7]: **G01N 33/543**, G01N 33/574

(21) Application number: **03015087.4**

(22) Date of filing: **03.07.2003**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(71) Applicant: **Labsoft Diagnostics AG**
**06108 Halle (DE)**

(72) Inventors:
• **Weber, Ekkehard**
**06124 Halle (DE)**
• **Günther, Dagmar**
**06193 Petersberg (DE)**
• **Zellmer, Sebastian**
**06118 Halle (DE)**

• **Goldberg, Martina**
**06110 Halle (DE)**
• **Bergmann, Christian**
**10823 Berlin (DE)**
• **Herrmann, Christine**
**06108 Halle (DE)**
• **Huske, Anita**
**06112 Halle (DE)**
• **Müller, Anja**
**06366 Köthen (DE)**

(74) Representative:
**Helbing, Jörg, Dr. Dipl.-Chem. et al**
**Patentanwälte**
**von Kreisler-Selting-Werner,**
**Postfach 10 22 41**
**50462 Köln (DE)**

(54) **Immunomagnetic separation of specific target cells**

(57) The present invention provides an advanced immunomagnetic method for the isolation of specific target cells from cell populations and suspensions of cell populations, especially tumor cells from peripheral blood bone, marrow aspirates, ascites and other body fluids. The invention also relates to a kit for performing the method for the isolation of specific target cells from body fluids.

EP 1 494 028 A1

## Description

The present invention provides an advanced immunomagnetic method for the isolation of specific target cells from cell populations and suspensions of cell populations, especially tumor cells from peripheral blood, bone marrow aspirates, ascites and other body fluids. The invention also relates to a kit for performing the method for the isolation of specific target cells, especially for the isolation of tumor cells from blood samples.

## Background of the Invention

Once a tumor gets malignant, tumor cells are disseminated into the blood and other compartments and spread into the body. These disseminated cells may give rise to metastases. The presence of disseminated tumor cells in blood, bone marrow aspirates and other body fluids is believed to be correlated with a decrease in survival. Therefore, the determination of disseminated tumor cells is of clinical interest and therapeutic importance. In addition the increase or decline in the number of disseminated tumor cells during therapeutic treatments will indicate the success of the treatment. Consequently, there is a medical need for the isolation and the characterization of disseminated tumor cells.

Disseminated tumor cells are characterized by the expression of specific cell surface structures (antigen-determinants, AG). These AG allow to distinguish between tumor cells and normal cells. AG can be identified by the use of polyclonal or monoclonal antibodies, since antibodies bind with high specificity.

Tumor-cell specific antibodies or molecules recognizing specific targets on tumor cells (e.g. aptamers) can be coupled onto paramagnetic particles (hereinafter also referred to as "beads"). If these antibody-bead complexes are incubated with suspensions, composed of tumor cells and other cells, a specific linkage between the tumor cells and the beads will occur.

Recently, EP-B-0660930 described a method for the detection of tumor cells using antibody-coated paramagnetic particles. However, in said method an incubation of the beads and/or the cell suspension with a mild detergent is necessary, since it will reduce the unspecific binding of non-target cells to the paramagnetic particles.

It has, however, to be kept in mind that individual tumor cells differ in their protein expression and consequently in the expression of cell surface structures (AG). Tumor cells with a low expression of AG, which are recognized by specific antibodies may not be detected. To circumvent this problem several solutions were proposed. First different antibodies directed against different AG of the tumor cells can be coupled onto the paramagnetic beads. Second, the bead surface can be modified in a way that a high attraction between tumor cells and beads exists. These forces can be reduced by the addition of mild detergents. Therefore, an incubation with detergents should be avoided in order to capture also tumor cells with a low expression of surface markers.

## Summary of the Invention

It was now found that paramagnetic particles which are loaded with antibodies/antibody fragments by a specific process avoid the above mentioned problems and are thus suitable for immunomagnetic isolation of target cells from body fluids without a preincubation of the cells with a detergent. Furthermore, it was found that the use of a specific buffer has an advantageous influence on immunomagnetic isolation utilizing such paramagnetic particles. The invention thus provides:

(1) a method for loading paramagnetic particles consisting of a core matrix containing paramagnetic material and having stably attached to its surface activatable functional groups capable of forming a chemical bond with nucleophilic groups on antibodies or antibody fragments, with antibodies or antibody fragments, which method comprises reacting particles having activated functional groups with antibodies or antibody fragments, and subsequently completely inactivating the remaining activated functional groups;

(2) a method for isolating and identifying specific target cells contained in body fluids, which comprises the steps of

(a)

(1) mixing paramagnetic particles loaded with first antibodies/antibody fragments directed against the target-cell specific membrane structures, or mixtures of said first antibodies/antibody fragments according to the method defined in (1) above with the body fluid containing the target-cells, or
(2) mixing and incubating free first antibodies/antibody fragments or mixtures of said first antibodies/antibody fragments with the body fluid containing the target cells;

(b)

(1) incubating the mixture obtained in step (a1), or
(2) mixing and incubating the mixture obtained in step (a2) with the paramagnetic particles loaded with second antibodies/ antibody fragments capable of binding to the Fc portion of said first antibodies/antibody fragments according to the method defined in (1) above;

(c) subjecting the mixture obtained in step (b) to a magnetic field to therewith separate the specific target cells from the mixture,

provided that steps (a) and (b) do not encompass a pre-incubation with detergents;
(3) a preferred embodiment of the method of (2) above, wherein the incubation step (b) is performed in an incubation buffer containing sugars, citric acid or a salt thereof, and lipids;
(4) a preferred embodiment of (3) above, wherein said incubation buffer comprises citric acid or a sodium or potassium salt thereof at a concentration of 2 to 20 mM, a mixture of hexoses and/or pentoses in a total concentration of 5 to 50 mM and lipids in a concentration of 0.01 to 10 g/l;
(5) paramagnetic particles loaded according to the method of (1) above;
(6) an incubation buffer as defined in (3) or (4) above;
(7) a kit for immunomagnetic isolation comprising the loaded paramagnetic particles of (5) above; and
(8) a preferred embodiment of the kit of (7) above, wherein the kit is suitable for performing the method of (2), (3) or (4) above and comprises

(i) paramagnetic particles loaded with first antibody/antibody fragments as defined in (2) above; or paramagnetic particles loaded with second antibodies/antibody fragments and free first antibodies/antibody fragments as defined in (2) above;
(ii) an incubation buffer as defined in (3) or (4) above.

The method (2), (3) and (4) of the invention is suitable to isolate, for diagnostic purposes, specific target cells from blood, bone marrow and other body fluids. It represents a sensitive isolation method for a variety of cell types, such that a high number of cells can be readily screened in the microscope or by FACS/FLOW analysis. The present method can be used for the isolation of cells for biochemical, molecular biological and immunological examinations. Also the isolated cells can be cultured and investigated further.

The method (2), (3) and (4) provides for the immunomagnetic isolation of target cells from a mixed cell population and physiological solutions containing cell populations and is suitable for positive isolation of specific types of both normal cells and abnormal cells. The method creates a linkage between a specific target cell and an insoluble support, such as paramagnetic particles, which consists of one or several elements. The particles are coated either with antibodies of murine, rat, bacterial, phage or other origin, directed to the specific antigen determinants in the membrane of the wanted target-cells, or the particles are coated with polyclonal or monoclonal antibodies capable of binding to the Fc portions of the specific anti-cell antibody directed to the antigen-determinants in the target-cell membranes. Target sensitive antibodies can be replaced by other target sensitive molecules like aptamers. Furthermore, the specificity of the test can be increased by adding a further set of antibodies or antibody fragments, prelabelled or not with fluorescent agents, metallocolloids, radioisotopes, biotin-complexes or certain enzymes allowing visualization or detection by other means.

## Detailed Description of the Invention

In the method (1) of the invention any core matrix suitable to form paramagnetic particles can be utilized. Preferably the core matrix is selected from silica, aluminum hydroxide, hydroxyapatite, zirconium hydroxide, etc.
Suitable paramagnetic material for preparing paramagnetic beads is well-known in the art. Any material having a positive but small magnetic susceptibility, due to the presence of atoms with permanent magnetic dipole moments, can be utilized. Particular material includes, but is not limited to, $MnSO_4$, $FeSO_4$, $CoCl_2$, $NiSO_4$, other salts of said metals, etc.
The activatable functional groups capable of forming a chemical bond with nucleophilic groups on the antibodies or antibody fragments can be functional groups which are directly activatable or are activatable through reaction with bifunctional reagents (which provide for an electrophilic site). Suitable activatable functional groups include, but are not limited to, -COOH, $-NH_2$, $-SO_3H$, -SH, CHO, -OH, acetals, epoxy groups or activated derivatives thereof, preferably are -COOH or activated derivatives thereof.
The activatable function groups can be attached to the surface of the paramagnetic material via a linker molecule carrying the activatable functional group. The linker molecule preferably comprises the structure

- X-fg

(wherein X is a $C_{1-20}$, preferably a $C_{3-8}$ alkylene group optionally interrupted by one to five heteroatoms including, but not limited to, -O-, -S-, -NH-, $-N(C_{1-3}$ alkyl)-, - S(O)- and $-S(O)_2$-, and fg is a functional group as defined above. Most preferably the linker comprises the structure $-(CH_2)_3NH(CH)_2COOH$ or $-(CH_2)_6COOH$. The attachment of the activatable functional groups via such linker molecule has the consequence that parts of the surface of the paramagnetic particles remain uncovered and are thus capable of participating in the binding of cells and the like. Particles carrying such linkers are commercially available as Sicastar-M (Micromod) and 75/H-TCL (Chemicell Sigma).
Alternatively, the functional groups can be attached to the particles via a natural or synthetic polymer coated on the paramagnetic particles, provided, however, that it provides the required functional groups. Preferably the polymer is selected from homopolymers or copolymers derived from monomers having unsaturated carbon chain and a functional group as defined hereinbefore, or a protected form thereof, preferably an acrylic acid or derivatives thereof.

The paramagnetic particles of the invention preferably have a diameter of about 0.5 to 2.5 $\mu$m, a density of about 1.5 to 3 g/cm$^3$, a functional group number of about 1 to 10 $\mu$mol COOH per g particle and/ or a degree of magnetization of about 1 to 10 emu/g.

In a particularly preferred embodiment the activatable functional group is a -COOH group attached to the surface of the particle via a linker molecule, preferably through the most preferred linker as defined above, the activation is effected by treatment with a carbodiimide (e.g. a water-soluble carbodiimide such as EDAC) and N-hydroxy succinimide and the inactivation of remaining activated -COOH groups is effected by treatment with ethanolamine, preferably at about pH 4 to 8, more preferably at about pH 5.5 to 7.8, even more preferred at about pH 7.40 to 7.45, most preferably at about pH 7.42.

In the method (2), (3) and (4) of the invention "avoiding of pre-incubation with amphiphiles" means that neither a separate pre-incubation step with detergents (which includes detergents, emulsifiers and the like) is performed nor are such detergents present in a significant amount (i.e. no more than 0.0001% v/v) in the solvent of the incubation steps (a) and (b), the entire absence of detergents in steps (a) and (b) being preferred.

In the method (2), (3) and (4) it is preferred that the body fluid prior to its mixing with the loaded particles or first antibodies/antibody fragments is subjected to dilution or ammonium chloride lysis. The lysis is preferably performed by incubating 1 ml peripheral blood, preferably plasma depleted, with 3 ml lysis solution containing $NH_4Cl$, $KHCO_3$ and EDTA (e.g. at concentrations of about 155 mM, 10 mM and 0.1 mM, respectively).

The first or second antibodies/antibody fragments or the beads may be labeled with functional moieties permitting their visualization by a physical or chemical reaction. If such functional moieties are not present on first or second antibodies/antibody fragments, it is preferred that prior to or after step (c) the reaction mixture is incubated with third antibodies or antibody fragments labeled with a functional moiety (e.g. an enzyme or the like), said third antibodies/antibody fragments being directed to extracellular or intracellular molecules present in the target cells, but differing from the membrane structures recognized by the first antibodies/antibody fragments. Suitable functional moieties are well-known in the art and include, but are not limited to, fluorescent agents, metallocolloids, radioisotopes, biotin-complexes or certain enzymes allowing visualization or detection by other means, among which fluorescent agents are most preferred.

In a particular preferred embodiment the counting of the stained or unstained partide-cell-complexes in the cell suspension is performed after step (c) by labeling with a target cell-specific third antibody carrying a fluorescent dye and by characterizing the particle-cell-complexes using a microscope and/or a suitable cell/particle counting device.

Moreover, it is preferred that the body fluids are derived from peripheral blood, bone marrow aspirates, from pleural or peritoneal effusions, urine, cerebrosipinal fluid, semen, lymph, from solid tumors in normal tissues and organs, preferably the body fluids are derived from peripheral blood or bone marrow aspirates, most preferably the body fluids are of human origin.

The specific target cells are selected from any one of primary abnormal cells, including tumor cells, metastatic tumor cells, disseminated tumor cells and the like. Preferably the target cells are selected from cells of breast cancer, ovarian cancer, lung carcinoma, melanoma, sarcoma, glioblastoma and other cancers, etc.

It is furthermore preferred that the first and second antibodies/antibody fragments are of the IgG, IgM, IgA, etc. isotype, deriving from e.g. mouse, rat, rabbit, goat, etd.. Particularly, the first antibodies/antibody fragments are monoclonal antibodies/antibody fragments, preferably are directed against groups of antigen determinants on the target cells, preferably against epithelial surface antigen (ESA), Her2/neu, melanocyte cell surface antigen, CD146, etc. The first antibody/antibody fragment can be replaced by other target sensitive molecules like aptamers, etc.. The second antibodies/antibody fragments are preferably polyclonal or monoclonal iodotypic antibodies or antibody fragments (e.g. anti-mouse, anti-rat, anti-rabbit, anti-goat, etc.) antibodies or antibody fragments.

In a further preferred embodiment methods (2), (3) and (4) of the invention furthermore include a wash step of the magnetic particles separated in step (c). Any wash solution not negatively influencing the separated cells can be used. It is however preferred that the wash solution is the incubation buffer, preferably composed as described herein below. The wash solution may be sterilized. The isolated cell can be examined by biochemical, molecular biological or immunological methods, preferably including a characterization of specific genes by identifying nucleic acids and proteins and/ or elucidating the structure of nucleic acids and proteins. It is also feasible that a culture of the isolated target cells or their complexes with the coated magnetic particles is established.

The incubation buffer used in embodiment (3) and (4) of the invention preferably contains citric acid or a sodium or potassium salt thereof at a concentration of 2 to 20 mM, a mixture of hexoses and/or pentoses in a total concentration of 5 to 50 mM and lipids in a concentration of 0.01 to 10 g/l. Particularly preferred incubation buffers are phosphate buffered saline containing citrate, a mixture of at least three hexoses and/or pentoses and lipids/liposomes at physiological pH. In more detail: citrate can be the free acid as well as any salt, preferably a sodium or potassium salt at a concentration of 2 to 20 mM, preferably at about 10.2 mM. The sugars are preferably hexoses, or their derivatives. Preferred is a mixture of D(+)glucose (about 5 to 30 mM, preferably about 18.6 mM), D(+)galactose (about 1 to 10 mM, preferably about 2.5 mM), D(+)mannose (about 1 to 10 mM, preferably about 2.5 mM) and L(-)fucose (about 1 to 10 mM, preferably about 2.5 mM). The lipid concentration is in the range of 0.01 to 100 g/l, preferably about 0.5 g/l. The lipids are composed of different phospholipids, mainly phosphatidylcholine, phosphatidylethanolamine tri- and diacylglycerols as well as lyso-lipids. The phospholipid concentration should be in the range of about 20-100 %, preferably at about 42% (basis total lipids). The main phospholipids should be phosphatidylcholine and/or phosphatidylethanolamine. The size of the particles formed by the lipids (liposomes) should be in the range of 50 nm to 5000 nm.

If the buffer is used only for the isolation and subsequent detection of tumour cells 0.02% sodium azide is added in order to increase shelf life. Alternatively the buffer can be filtrated sterile.

The incubation in the method (2), (3) and (4) is preferably performed at 0 to 37°C, preferably of about 4°C for 5 min to 2 h, preferably for about 30 min under gentle agitation.

In the following a more detailed disclosure of the method is presented, using cancer cells as the target-cells for detection and possible isolation. The method is, however, not limited to cancer cells and the disclosure shall not be construed to limit the method to this particular field of use, since the method is suitable within a range of cytological research areas.

In the management of cancer patients, the staging of the disease with regard to whether it is localized or if metastatic spread has occurred to other tissues, is of utmost importance for the choice of therapeutic alternatives for the individual patient. In addition, the number of detected tumor cells and the increase or decline in number during adjuvant therapy can give important information on the success of the therapy.

The tumor cells can be stained by immunohistochemistry using specific antibodies and examined by light (Cytotherapy 1(5), 377-388 1999) or fluorescence microscopy.

The invention allows for a very sensitive isolation and the subsequent characterization of, for example, metastatic tumor cells, since a high number of cells can readily be screened in the microscope. The monoclonal antibodies bind with sufficient specificity to, for example, tumor cells and not or to a much lower extend to other cells than the target-cells present in mixed cell suspensions, like blood or bone marrow.

Tumor cells vary in their expression of surface antigens. Therefore, a single type of antibody may not be sufficient to capture all target-cells. The isolation of target-cells is improved by binding two or more different types of target-cell specific antibodies/antibody fragments (first antibodies/antibody fragments) to the surface of the paramagnetic beads.

In addition, the surface properties of the beads are chosen in a way that binding of cells, especially tumor cells, selectively occurs. Therefore, tumor cells, which show only a minor expression of antibody binding target structures are captured by the beads.

The advanced method involves the coupling of monoclonal antibodies, e.g. of murine or other origin or generated by other biological methods (e.g. phage display), that specifically recognize antigens present on tumor cells, and not on normal cells in question to paramagnetic particles. For other purposes the antibodies are directed against specified subpopulations of normal cells. In a different approach the beads are coupled to antibodies specifically recognizing the tumor-specific antibodies. The cell binding antibodies may be of the IgG, IgA or IgM type or being a fragment of a IgG or IgM antibody. Examples of used anti-target-cell antibodies may be those directed against groups of antigen determinants, for example, epithelial surface antigen (ESA), Her2/neu (c-erbB2), melanocyte cell surface antigen or CD146. As for the malignant cells these may be breast, ovarian, and lung carcinoma cells, melanoma, sarcoma, glioblastoma, cancer cells of the gastrointestinal tract and the reticuloendothelial system. The malignant cell population may be located in bone marrow, peripheral blood, come from pleural and peritoneal effusions and other body fluid compartments, such as urine, cerebrospinal fluid, semen, lymph or from solid tumors in normal tissues and organs.

The method comprises coupling of the target-specific antibodies directly to the paramagnetic particles, or the attachment can take place by binding to surface coupled antibodies, such as mono- or polyclonal anti-mouse, anti-rat or other antibodies, specifically recognizing the Fc portion of the target-specific antibodies. The antibody-coated paramagnetic beads are then mixed with the suspension of cells to be isolated and incubated for 5-10 min to 2 h, preferably for 30 min at 0-25°C, preferably at 4°C, under gentle rotation in the presence of a solution or suspension containing sugars, citrate and lipids as defined hereinbefore.

The present method may also be performed in a different order of steps, in that free target-cell specific antibodies are added to the cell suspension, incubated for 5-10 min to 2 h, preferably 30 min, at 0-20°C, preferably 4°C, under gentle rotation and the paramagnetic particles - coated with anti-mouse or other antibodies - are then added to the incubated cell suspension and are incubated, as described above. The number of antibody coated beads added to the cell suspension should preferably be from about $5 \times 10^{-1}$ to $1 \times 10^{7}$, more preferably from about $1 \times 10^{1}$ to $1 \times 10^{6}$ times the number of target cells.

The target-cells can be positively separated from non-target cells in a magnetic field. The isolated target-cells, can then be enumerated microscopically and/or a suitable cell/particle counting device and the fraction of target cells relative to the total number of cells in the initial suspension can be calculated.

The target-cells may be characterized for the presence of specific biochemical and/or molecular biological features. Of particular importance will be the characterization of tumor cells present in blood, bone marrow and other biological fluids, for example, urine, cerebrospinal fluid, semen, and lymph by antibodies against target-cell specific intra- or extracellular markers.

If the material to be examined consists of blood or bone marrow aspirates, the erythrocytes are lysed (e. g. by the lysis buffer defined hereinbefore) and the remaining cells are used in the immunomagnetic isolation, described above.

The results of the immunomagnetic isolation are influenced by several factors. Among these are a) the ratio of target-cells to the number of particles, b) incubation times, type of incubation medium, type of antibodies and type of beads.

Individual tumor cells differ in their expression of specific antigens. Therefore, not all tumor cells are captured by the antibodies bound to the surface of the particles. In order to enhance the binding of tumor cells to antibody coated particles, particles are chosen that bind tumor cells by an unspecific mechanism. This can be demonstrated by the fact that beads free of any antibodies can bind a certain fraction of tumor cells. A pre-incubation of the antibody-coated beads or the cell suspension or both with a mild detergent (e.g. less than 0.1% Tween® 20) is in contrast to EP-B-0660930, not necessary. Rather, such preincubation with amphiphiles/detergents would reduce the number of isolated tumor cells (see Example 4).

After a immunomagnetic isolation, as described previously, the cell suspension can be incubated with a further set of antibodies or antibody fragments directed against other extracellular or against intracellular determinants of the target cells, with or without pretreatment with cell fixatives and/or permeabilization agents such as formaldehyde or alcohols. This second set of antibodies or their fragments should be prelabeled by fluorescent agents, metallocolloids, radioisotopes, biotin-complexes or enzymes like peroxidase and alkaline phosphatase, allowing visualization by per se known methods in the microscope and/or suitable counting device.

In order to simplify the characterization of isolated target-cells, the cell suspensions can be attached to coated glass slides or be subjected to cytospin centrifugation before the addition of the second set of antibodies.

The paramagnetic particles of the kit (7) of the invention are loaded with target-cell specific antibodies (e.g. a first antibodies specific for a tumor cell epitope) or with specific anti-Fc antibodies (second antibodies) such as polyclonal or monoclonal anti-mouse or other antibodies, capable of binding to the Fc-portion of the target-cell associated antibodies.

The kit (8) of the invention which is suitable to perform the method (2), (3) and (4) of the invention may further contain solutions and/or salts necessary for the lysis of erythrocytes from whole blood samples a wash solution for washing the cells by separation, a magnet, third antibodies as defined hereinbefore, and the like.

The invention is further explained by the following examples, which are however not to be construed to limit the invention.

**Examples**

1. Materials and Methods

LYSIS SOLUTION: The lysis solution is composed of 155 mM $NH_4Cl$, 10 mM $KHCO_3$, 0.1 mM EDTA and 0.02% sodium azide. The solution may be sterilized if necessary.

WASH SOLUTION: The wash solution is composed of 0.9% (w/v) NaCl with 0.6% sodium citrate and 0.02% sodium azide. The solution may be sterilized if necessary.

INCUBATION BUFFER

The incubation buffer is composed of phosphate buffered saline with 10.2 mM sodium citrate, 18.6 mM D(+)glucose, 2.5 mM D(+) galactose, 2.5 mM D(+) mannose, 2.5 mM L(-)fucose, 0.02 % sodium azide and 0.5 g/l soy-bead lipids with 42% (basis total lipids) phospholipids. The size of the particles formed by the lipids is below 5000 nm.

Paramagnetic silica particles purchased from Micromod (Sicastar-M; with terminal COOH-groups, 1.5 µm diameter, a density of 2.5 $g/cm^3$, a magnetization of 4 emu/g and a protein binding capacity of 1.4-1.6 µg Albumin/mg) or Chemicell Sigma (75/H-TCL with a terminal COOH-group, 0.75 µm diameter, a density of 2.25 $g/cm^3$ and a surface area of approximately 100 $m^2/g$).

M-buffer: 0.1 M MES buffer, pH 5.2
MT-buffer: 0.1 M MES buffer, pH 5.2, containing 0.01% Tween® 20
PBST: phosphate buffered saline containing 0.01% Tween® 20 at physiological pH, preferably pH 7.42.
PBSTE: PBST with 40 mM ethanolamine.

2. Preparation of CS-beads

Beads (2 µg) were resuspended in 1 ml 0.1 M MT-buffer and were washed by application of an external magnetic field for 5 min (alternatively the beads can be centrifuged). The supernatant was removed, the beads were resuspended in 1 ml MT-buffer and were then washed in 1 ml 0.1 M M-buffer.

The beads were activated by resuspension in 1 ml 0.1 M M-buffer containing 30 mg/ml EDAC and 6 mg/ml NHS for 15 min at room temperature under gentle agitation. The beads were then separated for 10 min in an external magnetic field, resuspended in 1 ml 0.1 M M-buffer, and washed twice in MT-buffer by application of a magnetic field as described above.

Antibody (10-40 µg/ml; e.g. purified mouse monoclonal antibodies BerEP4 (#09564 DakoCytometics) c-erbB-2 (#MS-229-PABX Neomarkers, Fremont, CA, USA) was added and incubated for 1 h at room temperature under gentle agitation. Then the beads were separated for 5 min in the presence of an external magnetic field as described above. Thereafter the beads were washed with PBST by application of a magnetic field as described above, were incubated for 30 min in 1 ml PBSTE and were then separated for 5 min in an external magnetic field. Finally, the beads were washed 3 times in PBST and were stored in 1 ml PBST at 4°C for further use.

3. Determination of the ratio of beads coated with antibodies

Beads coated with antibodies (e.g., the monoclonal mouse antibodies described in 2. above) were diluted 500-fold in PBST and separated for 5 min in an external magnetic filed. The beads were resuspended in 100 µl PBST and 2.5 µl anti-mouse F(ab)'$_2$ fragment of Ig antibody was added, which is fluorescence labeled with R/PE (#R0439 DakoCytometics). The beads and the antibodies were incubated for 30 min in the dark. Then 900 µl PBST were added and the beads separated in an external magnetic field for 5 min. The beads were washed in 1 ml PBST, resuspended in 1 ml sterile filtered PBS without Tween® and diluted 5-fold with sterile PBS. The relative amount of beads coated with antibody was determined by FLOW measurements. The beads were used if more than 70% of the beads were coated with antibodies.

4. Separation of cells

1 ml of a fresh EDTA-blood sample was filled into a conical 15 ml centrifuge tube and the sample was centrifuged at 1500 x g for 10 min at 4°C. The plasma was pipetted off without disturbing the buffy coat and the plasma volume was restored with the same volume of wash-solution. Then, 1 ml of LYSIS-SOLUTION was added and the cells were resuspended. 3 ml of LYSIS-SOLUTION was added and incubated at 2-8°C until erythrocyte lysis was complete (approx. 10 min). The color changed from bright red to deep red, sometimes almost black. The time of lysis was not increased since other cells might have been harmed. The mixture was centrifuged for 5 min at 4°C and 1500 x g and the supernatant was removed without disturbing the pellet. 1 ml of LYSIS-SOLUTION was added, the cells were resuspended and incubated for 10 min at 2-8°C. There was no visible change in the color. 9 ml of WASH-SOLUTION were added and centrifuged for 5 min at 4°C and 1500 x g. The supernatant was pipetted off and the cells were resuspended in 5 ml WASH-SOLUTION. For better results all cells were resuspended in 1 ml and the 4 ml was added. The mixture was centrifuged for 10 min at 4°C and 1500 x g and the supernatant was removed without disturbing the pellet. 1.5 ml INCUBATION-BUFFER was added and the suspension was transferred into a 2 ml round bottom vial. In order to reduce the loss of cells, the cells were first resuspended in 1 ml, then this volume was transferred into the 2 ml vial and the centrifuge tube was rinsed again with 0.5 ml INCUBATION-BUFFER. 15 µl CS-Beads, e.g. 2.5µl each of the beads prepared in 2. above) were added to the vial. The suspension was incubated on an end-over-end mixer at 4°C for 30 min at 20 rpm. The reaction tube was placed in the CS-Magnet for 10 min. The reaction tube was not moved or turned in the CS-Magnet. The liquid was pipetted off from the reaction tube without touching the beads. The beads and the rosetted cells were carefully resuspended in INCUBATION BUFFER.

5. Staining of separated cells

A fixation and permeabilization of the cells separated in 4. above was performed by using the FIX & PERM Cell Permeabilization kit from Caltag Laboratories (Hamburg, Germany).

100 µl solution A from the above kit were added and incubated for 15 min. This resulted in a fixation of the cells. The cells were washed in 3 ml phosphate buffered saline (PBS) with 1% fetal calf serum and 0.02% sodium azide (PBS/FCS). 100 µl solution B from the above kit of Caltag Laboratories were added, which permeabilized the cells and 2.5 µl FITC-labeled anti-Cytokeratin antibody (#130-080-101 Miltenyi Biotech GmbH, Bergisch Gladbach, Germany) and 2.5 µl R/PE-labeled anti-CD45 antibody (#IM2653 Beckman-Coulter, Krefeld, Germany) were added. The mixture was incubated for 15 min in the dark and the cells were washed in 3 ml PBS/FCS by centrifugation (400 x g, 10 min). The cells were resuspended in 1 ml PBS/FCS and the FLOW measurement was performed. The tumor cells were labeled with the above-mentioned anti-cytokeratin antibody.

6. Effect of a pre-incubation of antibody coated beads with INCUBATION BUFFER

Tumor cells (HCT15) were added to peripheral blood, which was lysed by a ammonium chloride method. Then beads were added, which were coated with an anti-ESA antibody (e.g. by a mixture of the beads prepared in 2. above). After an immunomagnetic separation as described in 4., 95% of the tumor cells were recovered. If the beads were pre-incubated with INCUBATION BUFFER, the recovery decreased to 65%, which shows that an incubation with amphiphilic substances (lipids) - contrary to detergents - provide for a reasonable recovery of tumor cells.

7. Binding of beads without antibodies to tumor cells

If the beads added were free of any antibody, then the recovery of the tumor cells (HCT15) was 12% without a pre-incubation and 11% with a pre-incubation in INCUBATION BUFFER. This shows that even the beads without antibodies specifically bind to tumor cells.

**Claims**

1. A method for loading paramagnetic particles consisting of a core matrix containing paramagnetic material and having stably attached to its surface activatable functional groups capable of forming a chemical bond with nucleophilic groups on antibodies or antibody fragments, with antibodies or antibody fragments, which method comprises reacting particles having activated functional groups with antibodies or antibody fragments, and subsequently completely inactivating the remaining activated functional groups.

2. The method according to claim 1, wherein

(i) the core matrix is selected from silica, aluminum hydroxide, hydroxyapatite, zirconium hydroxide, etc.; and/or
(ii) the paramagnetic material is selected from $MnSO_4$, $FeSO_4$, $CoCl_2$, $NiSO_4$, etc; and/or
(iii) the activatable functional groups capable of forming a chemical bond with nucleophilic groups on the antibodies or antibody fragments are directly activatable or through reaction with a bifunctional reagent, preferably said activatable functional groups are selected from -COOH, $-SO_3H$, $-NH_2$, -SH, CHO, -OH, acetals, epoxy groups or activated derivatives thereof, most preferably are - COOH or activated derivatives thereof; and/or
(iv) activatable functional groups are attached to the surface of the paramagnetic material via a linker molecule having the activatable functional group at its terminal end, preferably comprises the structure -X-tg, wherein X is a $C_{1-20}$ group optionally interrupted by one or more hteroatoms and tg is a functional group as defined hereinberfore, or via a coating with a natural and/or synthetic polymer carrying the activatable functional groups, preferably the natural or synthetic polymer is selected from homopolymers or copolymers derived from monomers having unsaturated carbon chain and a functional group as defined hereinbefore, or a protected form thereof, most preferably is an acrylic acid or derivatives thereof.

3. The method according to claim 1 or 2, wherein the activatable functional group is a -COOH group attached to the surface of the particle via a linker molecule, the activation is effected by treatment with a carbodiimide and N-hydroxy succinimide and the inactivation of remaining activated -COOH groups is effected by treatment with ethanolamine, preferably at pH 7.40 to 7.45.

4. A method for isolating and identifying specific target cells contained in body fluids, which comprises the steps of

(a)

(1) mixing paramagnetic particles loaded with first antibodies/antibody fragments directed against the target-cell specific membrane structures, or mixtures of said first antibodies/antibody fragments according to the method defined in any one of claims 1 to 3 with the body fluid containing the target-cells, or
(2) mixing and incubating free first antibodies/antibody fragments or mixtures of said first antibodies/antibody fragments with the body fluid containing the target cells;

(b)

(1) incubating the mixture obtained in step (a1), or
(2) mixing and incubating the mixture obtained in step (a2) with the paramagnetic particles loaded with second antibodies/antibody fragments capable of binding to the Fc portion of said first antibodies/antibody fragments according to the method defined in any one of claims 1 to 3;

(c) subjecting the mixture obtained in step (b) to a magnetic field to therewith separate the specific target cells from the mixture,

provided that steps (a) and (b) do not encompass a pre-incubation with amphiphiles.

5. The method of claim 4, wherein

(i) the body fluid prior to its mixing with the loaded particles or first antibodies/antibody fragments is subjected to dilution or ammonium chloride lysis, said lysis being preferably performed by adding a solution containing $NH_4Cl$,

KHCO$_3$ and EDTA; and/or

(ii) prior to or after step (c) the reaction mixture is incubated with third antibodies or antibody fragments labeled with functional moieties permitting their visualization by a chemical or physical reaction, preferably with enzymes, said third antibodies/antibody fragments being directed to extracellular or intracellular molecules present in the target cells, but differing from the membrane structures recognized by the first antibodies/antibody fragments, or the first or second antibodies/antibody fragments or the beads are labeled with functional moieties permitting their visualization by a chemical or physical reaction; and/or

(iii) the counting of the stained or unstained particle-cell-complexes in the cell suspension is performed after step (c) using a microscope and/or a suitable cell/particle counting device.

6. The method according to claim 4 or 5, wherein

(i) the body fluids are derived from peripheral blood, bone marrow aspirates, from pleural or peritoneal effusions, urine, cerebrosipinal fluid, semen, lymph, from solid tumors in normal tissues and organs, preferably the body fluids are derived from peripheral blood or bone marrow aspirates, most preferably the body fluids are of human origin; and/or

(ii) the specific target cells are selected from any one of primary abnormal cells, including tumor cells, metastatic tumor cells, disseminated tumor cells and the like, preferably are selected from cells of breast cancer, ovarian cancer, lung carcinoma, melanoma, sarcoma, glioblastoma and other cancers; and/or

(iii) the first and second antibodies/antibody fragments are of the IgG, IgM, IgA, isotype, preferably derived from mouse, rat, rabbit, goat, etc.; and/or

(iv) the first antibodies/antibody fragments are monoclonal antibodies/antibody fragments, preferably are directed against groups of antigen determinants on the target cells, preferably against epithelial surface antigen (ESA), Her2/neu, melanocyte cell surface antigen, CD146, etc.; and/or

(v) the second antibodies/antibody fragments are polyclonal or monoclonal iodotypic antibodies/antibody fragments, preferably anti-mouse, anti-rat, anti-rabbit or anti-goat antibodies or antibody fragments.

7. The method according to any one of claims 4 to 6, which further comprises

(i) a wash step of the magnetic particles separated in step (c); and/or

(ii) a step of examining the isolated cells by biochemical, molecular biological or immunological methods, preferably including a characterization of specific genes by identifying nucleic acids and proteins and/or elucidating the structure of nucleic acids and proteins; and/or

(iii) a step of establishing a culture of the isolated target cells or their complexes with the coated magnetic particles.

8. The method according to any one of claims 4 to 7, wherein

(i) the incubation step (b) is performed in an incubation buffer containing sugars, citric acid or a salt thereof and lipids, preferably said incubation buffer comprises citric acid or a sodium or potassium salt thereof at a concentration of 2 to 20 mM, a mixture of hexoses and/or pentoses in a total concentration of 5 to 50 mM and lipids in a concentration of 0.01 to 10 g/l; and/or

(ii) the incubation is performed at 0 to 37°C, preferably at about 4°C for 5 min to 2 h, preferably for about 30 min under gentle agitation.

9. Paramagnetic particles loaded with antibodies or antibody fragments according to the method of claims 1 to 3.

10. An incubation buffer as defined in claim 8.

11. A kit for immunomagnetic isolation comprising the loaded paramagnetic particles of claim 9.

12. The kit of claim 11 which is suitable for performing the method according to any one of claims 4 to 8, which comprises

(i) paramagnetic particles loaded with first antibody/antibody fragments as defined in any one of claims 4 to 6; or paramagnetic particles loaded with second antibodies/antibody fragments and free first antibodies/antibody fragments as defined in any one of claims 4 to 6;

(ii) an incubation buffer as defined in claim 8.

13. The Kit of claim 12 which further comprises

(iii) solutions and/or salts necessary for the lysis of erythrocytes in whole blood samples; and/or

(iv) wash solutions for washing the cells during separation; and/or

(v) a magnet; and/or

(vi) target-cell specific antibodies/antibody-fragments differing from the first antibodies/antibody fragments labeled with specific color detectable enzymes, such as peroxidase and alkaline phosphatase.

**European Patent Office**

**EUROPEAN SEARCH REPORT**

Application Number

EP 03 01 5087

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | GB 2 361 473 A (MICROGENICS CORP) 24 October 2001 (2001-10-24) * abstract * * page 6, line 11 - line 18 * * page 9, line 23 - page 10, line 5 * * page 13, line 22 - page 14, line 13 * * page 21, line 1 - line 19 * * table 1 * * page 32, line 4 - page 33, line 20 * | 1-3,9,11 | G01N33/543 G01N33/574 |
| X | WO 01 19956 A (BANDER NEIL H) 22 March 2001 (2001-03-22) * abstract * * page 1, line 1 - line 7 * * page 8, line 3 - line 29 * * page 9, line 1 - page 13, line 11 * | 1,4-7,9, 11 | |
| D,X  A | US 6 184 043 B1 (FODSTAD OSLASH YSTEIN ET AL) 6 February 2001 (2001-02-06) * the whole document * | 1-3,9,11  4-8,10, 12,13 | **TECHNICAL FIELDS SEARCHED** (Int.Cl.7) |
| X | US 4 554 088 A (JOSEPHSON LEE ET AL) 19 November 1985 (1985-11-19) * abstract * * column 11, paragraph 6 - column 17 * * column 17, paragraph 7 - column 27 * | 1-3,9,11 | G01N |

-/--

The present search report has been drawn up for all claims

| Place of search | Date of completion of the search | Examiner |
|---|---|---|
| THE HAGUE | 17 December 2003 | Vanhalst, K |

# EP 1 494 028 A1

**European Patent Office**

**EUROPEAN SEARCH REPORT**

**Application Number**

EP 03 01 5087

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 01 67105 A (STOHR UWE ;WIEGAND ANDREAS (DE); KIRAKOSSIAN HRAIR (US); PEASE JOH) 13 September 2001 (2001-09-13) * abstract * * page 6, line 20 - line 27 * * page 9, line 9 - line 11 * * page 11, line 30 - page 12, line 7 * * page 13, line 16 - line 28 * * page 14, line 25 - page 16, line 15 * * page 16, line 26 - page 17, line 30 * * page 18, line 21 - page 20, line 5 * * page 21, line 26 - page 22, line 21 * * page 28, line 20 - page 28, line 25 * * page 36, line 27 - page 37, line 6 * * page 48; example K * | 1-3,9,11 | |
| A | WO 90 04019 A (BAXTER INT) 19 April 1990 (1990-04-19) * abstract * * example 1 * * page 18, line 20 - page 22, line 20 * | 1-7 | |
| A | WO 01 35759 A (HERR JOHN C ;UNIV VIRGINIA (US); DIEKMAN ALAN B (US); KLOTZ KENNET) 25 May 2001 (2001-05-25) * abstract * * page 4, line 1 - page 7, line 12 * | 1-6,9 | TECHNICAL FIELDS SEARCHED (Int.Cl.7) |
| A | WO 03 042699 A (ACLARA BIOSCIENCES INC) 22 May 2003 (2003-05-22) * abstract * | 1-6 | |

The present search report has been drawn up for all claims

| Place of search | Date of completion of the search | Examiner |
|---|---|---|
| THE HAGUE | 17 December 2003 | Vanhalst, K |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 03 01 5087

This annex lists the patent family members relating to the patent documents cited in the above-mentioned European search report.
The members are as contained in the European Patent Office EDP file on
The European Patent Office is in no way liable for these particulars which are merely given for the purpose of information.

17-12-2003

| Patent document cited in search report | | Publication date | Patent family member(s) | | Publication date |
|---|---|---|---|---|---|
| GB 2361473 | A | 24-10-2001 | DE | 10111224 A1 | 21-02-2002 |
| | | | US | 2003207469 A1 | 06-11-2003 |
| WO 0119956 | A | 22-03-2001 | AU | 7351800 A | 17-04-2001 |
| | | | EP | 1221053 A2 | 10-07-2002 |
| | | | WO | 0119956 A2 | 22-03-2001 |
| | | | US | 6653129 B1 | 25-11-2003 |
| US 6184043 | B1 | 06-02-2001 | WO | 9407138 A1 | 31-03-1994 |
| | | | AT | 186402 T | 15-11-1999 |
| | | | AU | 2593192 A | 12-04-1994 |
| | | | AU | 686569 B2 | 12-02-1998 |
| | | | AU | 4836393 A | 12-04-1994 |
| | | | CA | 2144328 A1 | 31-03-1994 |
| | | | CZ | 9500659 A3 | 15-11-1995 |
| | | | DE | 69326956 D1 | 09-12-1999 |
| | | | DE | 69326956 T2 | 15-06-2000 |
| | | | DK | 660930 T3 | 08-05-2000 |
| | | | EP | 0660930 A1 | 05-07-1995 |
| | | | ES | 2141170 T3 | 16-03-2000 |
| | | | FI | 951161 A | 09-05-1995 |
| | | | GR | 3032547 T3 | 31-05-2000 |
| | | | HU | 73741 A2 | 30-09-1996 |
| | | | JP | 3417563 B2 | 16-06-2003 |
| | | | JP | 8501390 T | 13-02-1996 |
| | | | NO | 950918 A | 10-05-1995 |
| | | | WO | 9407139 A1 | 31-03-1994 |
| | | | PL | 308109 A1 | 24-07-1995 |
| | | | PT | 660930 T | 28-04-2000 |
| | | | SK | 32995 A3 | 11-10-1995 |
| US 4554088 | A | 19-11-1985 | AT | 70366 T | 15-12-1991 |
| | | | CA | 1254028 A1 | 16-05-1989 |
| | | | CA | 1266769 A2 | 20-03-1990 |
| | | | DE | 3485332 D1 | 23-01-1992 |
| | | | DK | 237484 A | 13-11-1984 |
| | | | EP | 0125995 A2 | 21-11-1984 |
| | | | EP | 0357593 A1 | 14-03-1990 |
| | | | JP | 2113602 C | 06-12-1996 |
| | | | JP | 7006986 B | 30-01-1995 |
| | | | JP | 60001564 A | 07-01-1985 |
| | | | JP | 2683786 B2 | 03-12-1997 |
| | | | JP | 8009995 A | 16-01-1996 |
| | | | WO | 8806632 A1 | 07-09-1988 |
| | | | US | 4628037 A | 09-12-1986 |
| | | | US | 4695392 A | 22-09-1987 |

EPO FORM P0459

For more details about this annex : see Official Journal of the European Patent Office, No. 12/82

**EP 1 494 028 A1**

**ANNEX TO THE EUROPEAN SEARCH REPORT
ON EUROPEAN PATENT APPLICATION NO.**

EP 03 01 5087

This annex lists the patent family members relating to the patent documents cited in the above-mentioned European search report.
The members are as contained in the European Patent Office EDP file on
The European Patent Office is in no way liable for these particulars which are merely given for the purpose of information.

17-12-2003

| Patent document cited in search report | | Publication date | Patent family member(s) | | Publication date |
|---|---|---|---|---|---|
| US 4554088 | A | | US 4695393 A | | 22-09-1987 |
| | | | US 4698302 A | | 06-10-1987 |
| | | | US 4672040 A | | 09-06-1987 |
| WO 0167105 | A | 13-09-2001 | WO 0167105 A1 | | 13-09-2001 |
| | | | CA 2400993 A1 | | 13-09-2001 |
| | | | EP 1264181 A1 | | 11-12-2002 |
| | | | JP 2003526786 T | | 09-09-2003 |
| WO 9004019 | A | 19-04-1990 | AU 4492789 A | | 01-05-1990 |
| | | | CA 1335181 C | | 11-04-1995 |
| | | | DE 68917736 D1 | | 29-09-1994 |
| | | | DE 68917736 T2 | | 04-05-1995 |
| | | | EP 0438520 A1 | | 31-07-1991 |
| | | | JP 7057177 B | | 21-06-1995 |
| | | | JP 4500008 T | | 09-01-1992 |
| | | | WO 9004019 A1 | | 19-04-1990 |
| | | | US 5536475 A | | 16-07-1996 |
| WO 0135759 | A | 25-05-2001 | AU 1778701 A | | 30-05-2001 |
| | | | WO 0135759 A1 | | 25-05-2001 |
| | | | US 2002182751 A1 | | 05-12-2002 |
| WO 03042699 | A | 22-05-2003 | WO 03042699 A1 | | 22-05-2003 |
| | | | US 2003134333 A1 | | 17-07-2003 |

EPO FORM P0459

For more details about this annex : see Official Journal of the European Patent Office, No. 12/82

14